# EUROPÄISCHE PATENTSCHRIFT

(11) **EP 1 033 365 B1**
(45) Veröffentlichungstag und Bekanntmachung des Hinweises auf die Patenterteilung: **25.09.2002**
(21) Anmeldenummer: 00103941.1
(22) Anmeldetag: 25.02.2000
(51) Int. Cl.: C07C 307/02, C05C 9/00, C05F 11/00

(54) **Diureide und deren Verwendung**
Diureides and their use
Composé de diuréides et leur utilisation

(30) Priorität: 03.03.1999 DE 19909334; 02.12.1999 DE 19958030
(43) Veröffentlichungstag der Anmeldung: 06.09.2000
(73) Patentinhaber: BASF AKTIENGESELLSCHAFT, 67056 Ludwigshafen (DE)
(72) Erfinder: Horchler von Locquenghien, Klaus, Dr., 67117 Limburgerhof (DE); Erhardt, Klaus, Dr., 69181 Leimen (DE); Weigelt, Wolfgang, 67373 Dudenhofen (DE); Dressel, Jürgen, Dr., 67141 Neuhofen (DE); Wissemeier, Alexander, Dr., 67346 Speyer (DE); Boerner, Frank, 12203 Berlin (DE); Kossmehl, Gerhard, Prof.Dr., 12209 Berlin (DE)

(56) Entgegenhaltungen:
- US-A- 2 090 594
- US-A- 3 459 529

## Beschreibung

Die vorliegende Erfindung betrifft Düngemittel wie Stickstoffdüngemittel, insbesondere bekannte Diureide sowie neue Diureide, ein Verfahren zu ihrer Herstellung sowie ihre Verwendung als Düngemittel mit Langzeitwirkung in Landwirtschaft und Gartenbau.

Aus der DD-A-264 372 und der WO-A-87/05781 sind ähnliche Malonsäurederivate zur Verzögerung des Pflanzenwachstums, aus der WO-A-87/05897 ähnliche Malonsäurederivate zur Steigerung des Ernteertrags bekannt. Die dort beschriebenen Verbindungen werden nur in relativ niedrigen Aufwandmengen eingesetzt und beziehen ihre Wirkung aus ihrer Eigenschaft als Wachstumsregulatoren, die das Pflanzenwachstum reduzieren.

Aus Chem. Ber. 1913, 46, Seite 1408 ist (R = M; X = Einfachbindung) Oxalsäurediureid, aus EP-A-254 683 ist (R = H, X = CH₂) Malonsäurediureid, aus J. Prakt. Chem. 1874, (2) 9, Seite 301 ist (R = H, X = CH₂CH₂) Bernsteinsäurediureid, aus US-A-2,090,594 ist (R = H, X = CH₂CH₂CH₂) Glutarsäurediureid und aus Chem. Zentralbl. 1936, 107 (I), Seite 1218 ist (R = H, X = CH = CH) Fumarsäurediureid bekannt.

Diese Verbindungen als solche und ihre Synthese sind bekannt; ihre Eignung und Verwendung als Düngemittel ist jedoch neu.

Der vorliegenden Erfindung lag die Aufgabe zugrunde, chemische Verbindungen zu finden, die als Langzeitdünger geeignet und den bekannten Substanzen im Bereich Pflanzenverträglichkeit und Wirkungsdauer überlegen sind. Ferner sollte es möglich sein, daß mehrere Pflanzennährstoffe in den Langzeitdüngern enthalten sind und verzögert freigesetzt werden.

Demgemäß wurde die Verwendung von Diureiden von Dicarbonsäuren der allgemeinen Formel I 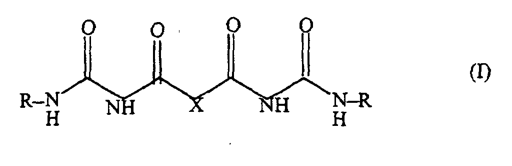 in der
- R: Wasserstoff oder SO₂O^{⊖}M^{⊕,}
- M^{⊕}: Lithium, Natrium, Kalium, Cäsium, Ammonium, Kupfer, Silber, 0,5 Eisen, 0,5 Calcium, 0,5 Magnesium, 0,5 Mangan, 0,5 Zink oder 0,5 Cobalt und
- X: eine gesättigte oder einfach ungesättigte, gegebenenfalls durch Sauerstoff oder NH unterbrochene geradkettige oder verzweigte, gegebenenfalls C₁- bis C₄-Alkoxy, Hydroxy und/oder Aminogruppen tragendes C₁-bis C₈-Alkyl bedeutet,
als Langzeitdüngemittel gefunden sowie neue Diureide der allgemeinen Formel Ia 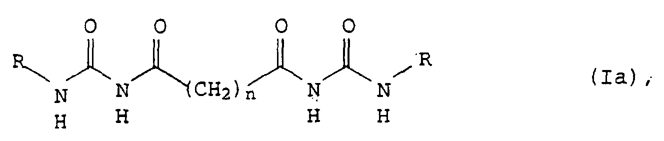 in der
- R: SO₂O^{⊖}M^{⊕,}
- M^{⊕}: Lithium, Natrium, Kalium, Cäsium, Ammonium, Kupfer, Silber, 0,5 Eisen, 0,5 Calcium, 0,5 Magnesium, 0,5 Mangan, 0,5 Zink oder 0,5 Cobalt und
- n: 1, 2, 3 oder 4
bedeuten, gefunden sowie ein Verfahren zu deren Herstellung.

Die erfindungsgemäßen Verbindungen Ia lassen sich wie folgt herstellen:

Man kann zu Malonsäurediamid in einem geeigneten Lösungsmittel mit Chlorsulfonylisocyanat bei einer Temperatur von (-20) bis +190°C, bevorzugt (-15) bis + 50°C, besonders bevorzugt (-10) bis 0°C, versetzen. Die entstehende Mischung kann auf Eis gegossen werden und anschließend mit einer M^{⊕}₂-Carbonatlösung, bevorzugt mit einer konzentrierten wäßrigen M^{⊕}₂-Carbonatlösung bei einer Temperatur von (-10) bis +50°C, bevorzugt 0 bis 30°C, besonders bevorzugt bei Raumtemperatur (18 bis 28°C) versetzt werden.

Als geeignete Lösungsmittel können übliche aprotische Lösungsmittel, beispielsweise Ether wie Diethylether, Methyl-tert.-butylether oder aromatische Kohlenwasserstoffe wie Benzol, Toluol und die Xylole, oder Nitrile wie Acetonitril und Propionitril, oder Ketone wie Aceton, Ethylmethylketon und Diethylketon, Ester wie Ameisensäuremethylester, Ameisensäureethylester, Essigsäuremethylester und Essigsäureethylester, Sulfoxide wie Dimethylsulfoxid oder Formamide wie Dimethylformamid, Ethylmethylformamid und Diethylformamid oder deren Gemische verwendet werden, bevorzugt werden aprotische Lösungsmittel, die sich mit Wasser mischen, besonders bevorzugt sind Acetonitril, Aceton, Essigmethylester und Dimethylformamid.

Die Herstellung kann sowohl kontinuierlich als auch diskontinuierlich erfolgen.

Die Verbindungen sind überraschend schwerlöslich in Wasser und eignen sich daher als Quelle für Pflanzennährstoffe mit Langzeitwirkung. Im Boden unterliegen sie einer langsamen Mineralisation (s.u.), wobei nach und nach alle enthaltenen Nährstoffe pflanzenverfügbar werden. Im Gegensatz zu den o.b. üblichen Langzeitdüngemitteln sind dies bei den erfindungsgemäßen Verbindungen neben Stickstoff auch Schwefel und (je nach Gegenion M^{⊕}) Kalium, Calcium, Magnesium, Eisen, Kupfer, Zink oder Mangan. Insbesondere der mögliche Einsatz der Verbindungen als kombinierte Nährstoffquelle mit slow release-Eigenschaften für Hauptnährstoffe wie N und K, Sekundärnährstoffe wie Schwefel, Magnesium und Calcium sowie Spurennährstoffe wie Eisen, Kupfer, Mangan, Zink macht die Verbindungen interessant.

Die erfindungsgemäßen neuen Diureide I lassen sich in relativ hohen Mengen pro Hektar einsetzen und führen zu Ertragsverbesserungen durch Steigerung des Pflanzenwachstums. Die in den Substanzen enthaltenen Pflanzennährstoffe werden mit der Zeit pflanzenverfügbar und entfalten daher eine Wirkung als Langzeitdüngemittel.

Düngemittel mit Langzeitwirkung bieten gegenüber konventionellen mineralischen oder organischen Düngemitteln viele Vorteile. Sie bieten eine bedarfsgerechtere Anlieferung der Nährstoffe an die Pflanze und verbessern dadurch die Nährstoffausnutzung. Dies führt zu einer Verringerung von Nährstoffverlusten, wodurch die Umweltbelastung reduziert und die Effizienz der Düngung gesteigert wird. Außerdem erlauben sie es Arbeitsgänge und Betriebsmittel und somit agrarwirtschaftliche Kosten einzusparen.

Eine Langzeitwirkung von Düngemitteln kann auf unterschiedlichen Wegen erreicht werden. Eine Möglichkeit ist in Wasser leichtlösliche granulierte Düngemittel mit einer wasserunlöslichen Hülle zu umgeben. Die Nährstofffreisetzung aus solchen umhüllten Düngemitteln erfolgt verzögert, da die Nährstoffe erst durch die Hüllschicht hindurchdiffundieren müssen bevor sie von den Wurzeln aufgenommen werden können. Eine andere Möglichkeit besteht darin, die Nährstoffe in Form von chemischen Verbindungen auszubringen, in der sie zunächst nicht pflanzenverfügbar sind. Erst nach Ablauf eines vorgelagerten Freisetzungsschrittes, z.B. einer chemischen Hydrolyse, einer enzymatischen Spaltung und/oder einer mikrobiellen Umsetzung, liegen die Nährstoffe in einer für die Pflanzen verwertbaren Form vor. Die hier beschriebene Erfindung zählt zu dieser zweiten Gruppe von Düngemitteln, den chemischen Langzeitdüngern. Beispiele für solche chemischen Langzeitdünger sind Harnstoff-Aldehyd-Kondensate wie Isodur (DE-A-32 41 445) oder Ureaform (WO-A-97/05084).

Die erfindungsgemäßen Verbindungen I können für sich allein oder als Mischungen bzw. in Kombination mit anderen üblichen Düngemitteln oder Zusätzen als Langzeitdünger eingesetzt werden. Sie lassen sich z.B. mit üblichen Kalidüngemitteln (K-Düngemitteln) wie Kaliumchlorid, Kaliumsulfat und Kaliumnitrat, Stickstoffdüngemitteln (N-Düngemitteln) wie Kalkammonsalpeter, Ammoniumsulfat, Ammoniumnitrat, Ammonsulfatsalpeter und Harnstoff, Stickstoffphosphordüngemitteln (NP-Düngemitteln) wie Ammoniumphosphate, Stickstoffkaliumdüngemitteln (NK-Düngemitteln) wie Kaliumammoniumsulfat, Phosphorkaliumdüngemitteln (PK-Düngemitteln) wie Kaliumphosphat oder Stickstoffphosphorkaliumdüngemitteln (NPK-Düngemitteln) wie Kaliumammoniumphosphat, die gegebenenfalls Langzeitwirkung haben können, zusammen formulieren.

Neben den genannten Hauptbestandteilen kann ein erfindungsgemäßes Düngemittel noch Sekundärnährstoffe wie Calcium, Schwefel und/oder Magnesium und Spurenelemente wie Bor, Eisen, Mangan, Kupfer, Zink und/oder Molybdän in untergeordneten Mengen, d.h. üblicherweise in Mengen von 0,5 bis 5 Gew.-% sowie weitere Zusatzstoffe wie Pflanzenschutzmittel, z.B. Insektizide, Herbizide oder Fungizide, Wachstumsregulatoren oder Nitrifikationsinhibitoren enthalten.

Sie können zur Anwendung in bekannter Weise, z.B. durch Granulieren oder Kompaktieren, auf eine gewünschte Korngröße gebracht werden. Derartig formulierte Düngemittel weisen im allgemeinen einen längsten mittleren Teilchendurchmesser von 0,5 bis 10 mm, bevorzugt 0,7 bis 5 mm auf. Ihr Schüttgewicht liegt üblicherweise bei 0,5 bis 1,3 kg/l.

Die erfindungsgemäßen Düngemittel werden üblicherweise nach den allgemein bekannten Verfahren auf landwirtschaftlich und gärtnerisch genutzte Flächen aufgebracht (vgl. Ullmann's Encyclopädie of Industrial Chemistry, 5.Auflage 1987, Band A 10, Seiten 398 bis 401) oder in Topf- oder Containerkulturen bzw. Pflanzsubstrate eingemischt. Wegen ihrer hohen Pflanzenverträglichkeit eigenen sich die erfindungsgemäßen Granulatmischungen nicht nur für Düngeverfahren, bei denen der Dünger annähernd gleichmäßig auf die Agrarfläche verteilt wird, sondern für die gezielte Ablage in der Nähe der Pflanzenwurzel. Die Aufwandmengen liegen im allgemeinen zwischen 1 und 5000 kg/ha, besonders günstig zwischen 20 und 1800 kg/ha.

Die erfindungsgemäßen Düngemittel können grundsätzlich in allen Bereichen des Pflanzenbaus wie der Landwirtschaft und dem Gartenbau eingesetzt werden, vor allem dem Obst- und Gemüsebau. Kulturen, deren Wachstum mit dem erfindungsgemäßen Düngemittel darüber hinaus effizient gefördert werden können, sind zum Beispiel Mais, Baumwolle, Süßkartoffeln, Weißkartoffeln, Luzerne, Weizen, Roggen, Reis, Gerste, Hafer, Hirse, Trockenbohnen, Sojabohnen, Zuckerrüben, Sonnenblumen, Tabak, Hopfen, Tomaten, Canola, abfallende Früchte, Zitrusfrüchte, Tee, Kaffee, Oliven, Ananas, Kakao, Bananen, Zuckerrohr, Ölpalmen, krautartige Freilandpflanzen, holzige Sträucher, Rasengräser, Zierpflanzen, immergrüne Pflanzen, Bäume, Blumen und dergleichen.

Die erfindungsgemäßen Düngemittel zeichnen sich dadurch aus, daß sie eine besonders wirtschaftliche intensive Nutzung von landwirtschaftlich und gärtnerisch genutzten Flächen ermöglichen, wobei eine Belastung der Umwelt weitgehend vermieden wird.

Für einen optimale Wachstumsförderung der Pflanzen reicht es im allgemeinen aus, die erfindungsgemäßen Düngemittel einmal pro Wachstumsperiode (bevorzugt zu deren Beginn) auf die Agrarfläche aufzubringen, weil der Nährstoffbedarfsverlauf der Pflanzen und der Freisetzungsverlauf der Düngemittelwirkstoffe weitgehend übereinstimmen.

Die Reste R, R', X und der Index n in den Formeln I und Ia haben folgende Bedeutungen:
- R: Wasserstoff
R,R' SO₂O^{⊖}M^{⊕},
- M^{⊕}: Lithium, Natrium, Kalium, Cäsium, Ammonium, Kupfer, Silber, 0,5 Eisen, 0,5 Calcium, 0,5 Magnesium, 0,5 Mangan, 0,5 Zink oder 0,5 Cobalt, bevorzugt Natrium, Kalium, Ammonium, 0,5 Eisen, 0,5 Mangan, 0,5 Magnesium oder 0,5 Zink, besonders bevorzugt Natrium, Kalium oder 0,5 Magnesium,
- X: eine ungesättigte oder einfach ungesättigte, gegebenenfalls durch Sauerstoff oder NH unterbrochene geradkettige oder verzweigte, gegebenenfalls C₁- bis C₄-Alkoxy, bevorzugt Methoxy oder Ethoxy, besonders bevorzugt Methoxy, Hydroxyl und/oder Aminogruppen tragendes C₁- bis C₈-Alkyl, bevorzugt C₁- bis C₅-Alkyl, besonders bevorzugt Methyl, Ethyl, n-Propyl, iso-Propyl, n-Butyl, iso-Butyl, sec.-Butyl und tert.-Butyl,
- n: 1, 2, 3 und 4, bevorzugt 1, 2 und 3, besonders bevorzugt 1 und 2.

### Ausführungsbeispiel

Die Erfindung wird durch die folgenden Beispiele veranschaulicht.

Zur Demonstration der Wirkung dieser Substanzen wurden umfangreiche pflanzenbauliche Versuche durchgeführt. Hierzu wurden Versuche mit Weidelgras als Prüfkultur in Mitscherlichgefäßen angelegt und das Gras in regelmäßigen Abständen geschnitten, seine Trokkenmasse bestimmt und auf seinen N-Gehalt analysiert. Außerdem wurden die Pflanzen vor jedem Schnitt bewertet und auf Schädigungen hin untersucht. Die Stickstoffgabe von 2,4 g N pro Gefäß lag mit auf die Gefäßoberfläche umgerechneten 770 kg N/ha deutlich über der praxisüblich eingesetzten Düngermenge, so daß bei einigen der zum Vergleich eingesetzten Standarddüngemittel wie Harnstoff oder Ammoniumnitrat die Verträglichkeitsgrenze überschritten war und Pflanzenschäden provoziert wurden.

Die folgenden Beispiele erläutern Vorteile der Erfindung. Die Beispiele 1 bis 5 sind Vergleichsbeispiele; die Beispiele 6 bis 9 sind Ausführungsformen der Erfindung. Beispiel 1 ist ein Versuch ohne Düngerzusatz; die Beispiele 2 und 3 sind Versuche mit Düngemitteln ohne Langzeitwirkung. Die Beispiele 4 und 5 wurden mit handelsüblichen Langzeitdüngern durchgeführt. In den Beispielen 6 bis 8 wurden 3 erfindungsgemäße Langzeitdünger eingesetzt. Im Beispiel .9 wurde eine Mischung aus Malonsäurediureid und Ammoniumnitrat 1:1 verwendet. Während bei den Beispielen 4 und 5 die Wirkung bereits nach 2 bis 3 Monaten deutlich nachließ, zeigten die Beispiele 6 bis 8 über einen Zeitraum bis zu mehr als 8 Monaten eine sehr gleichmäßige Stickstoffanlieferung und Stickstoffaufnahme und keine Pflanzenschäden. Das Beispiel 9 ergab eine stärker betonte Anfangswirkung bei ebenfalls sehr guter Verträglichkeit.

Die in den Tabellen 1 und 2 dargestellte Trockenmasseproduktion beziehungsweise Stickstoffaufnahme zeigt bei den Vergleichsbeispielen nur etwa bis zum 100. Tag nach der Zugabe eine nennenswerte Wachstumsförderung, während die erfindungsgemäßen Beispiele noch bis zum 125. Tag und - soweit Messungen gemacht wurden - bis zum 252. Tag eine deutliche Stickstoffaufnahme und damit Trockenmasseproduktion erkennen lassen. Die erfindungsgemäß eingesetzten Substanzen wirken also als Langzeitdüngemittel mit einer Wirkungsdauer von etwa 6 bis 9 Monaten.

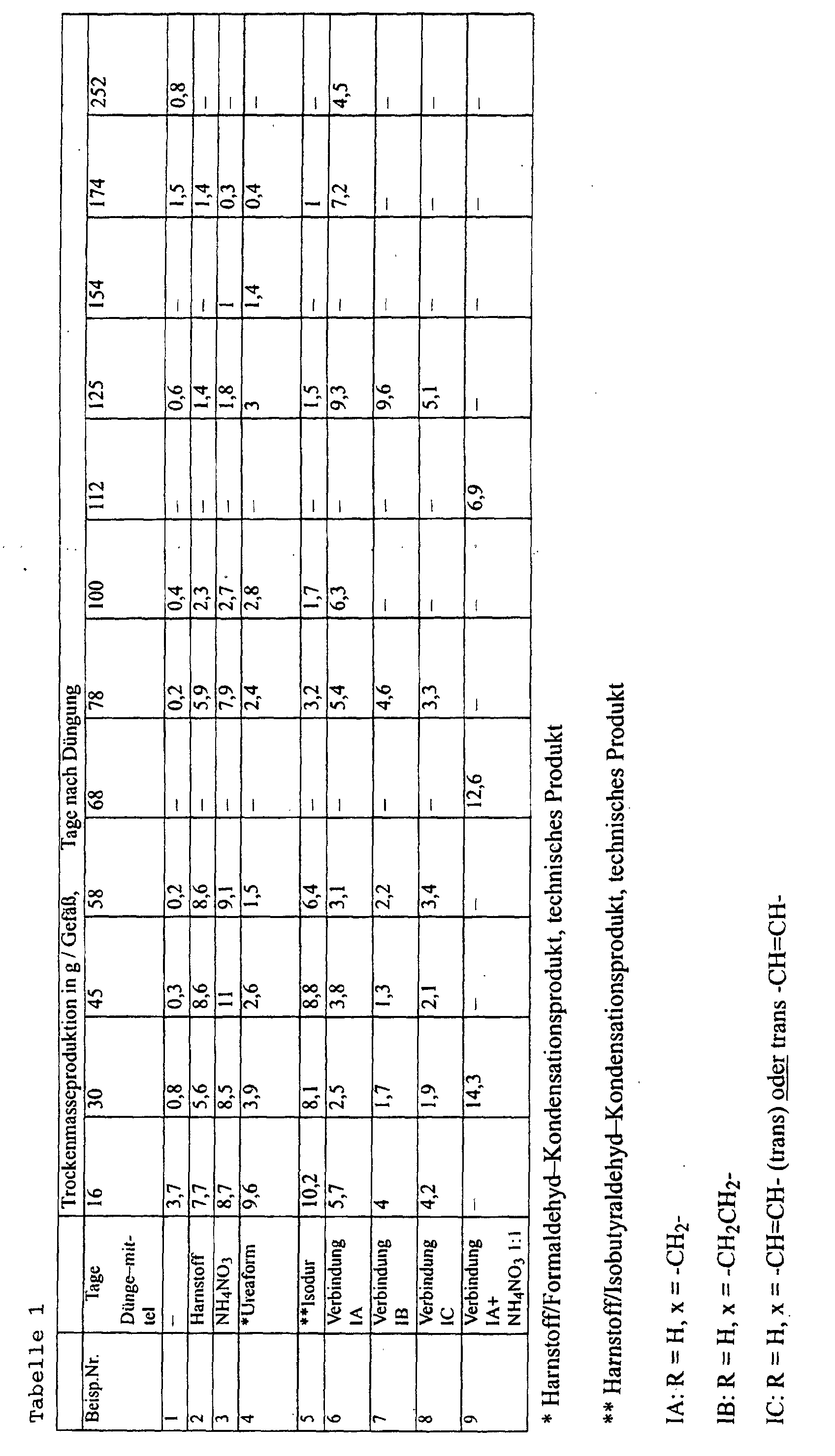

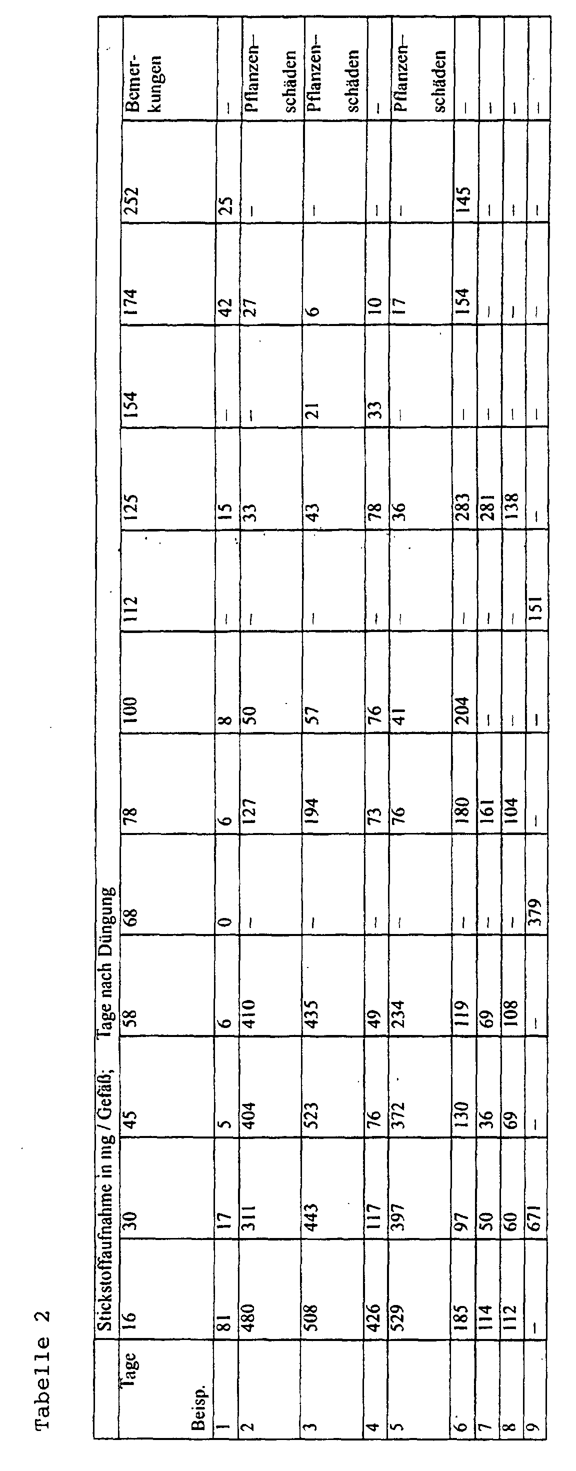

### Beispiel I

### Herstellung von Bis-(N'-Kaliumsulfamat)-malonsäurediureid

51 g (0,5 mol) Malondiamid wurden bei 70°C i.Vak. 30 min getrocknet, mit 100 ml abs. Acetonitril (getrocknet über Molekularsieb) aufgeschlämmt, auf (-10°C) gekühlt, 149 g (1,05 mol) Chlorsulfonylisocyanat bei einer Temperatur zwischen (-3) und (-10°C) zugetropft und 2 h bei Raumtemperatur nachgerührt. Der weiße Kristallbrei wurde auf 2 kg Eis gegossen, 138,2 g (1 mol) Kaliumcarbonat als konzentrierte Wasserlösung zugegeben und der entstehende weiße Niederschlag abgesaugt, mit 0,5 l Wasser und dann 0,5 l Aceton gewaschen und getrocknet. Man erhielt 157 g (74 %) Bis-(N⁺-Kaliumsulfamat)malonsäurediureid; Zersp.: 210°C, weißes Pulver.

### Herstellung von Bis-(N'-Kaliumsulfamat)-succindiureid

5 g (43 mmol) Succindiamid wurden bei 70°C i. Vak. 30 min getrocknet, mit 100 ml abs. Acetonitril (getrocknet über Molekularsieb) aufgeschlämmt, auf (-10°C) abgekühlt, 12,4 g (88 mmol) Chlorsulfonylisocyanat bei einer Temperatur zwischen (-3) und (-10°C) zugetropft und 2 h bei Raumtemperatur nachgerührt. Der weiße Kristallbrei wurde auf ca. 2 kg Eis gegossen, 12,2 (88 mmol) Kaliumcarbonat als konzentrierte Wasserlösung zugegeben und der entstehende weiße Niederschlag abgesaugt, mit 0,5 l Wasser, dann mit 0,5 1 Aceton gewaschen und getrocknet. Man erhielt 117 g (62 %) Bis-(N'-Kaliumsulfamat)succindiureid; Schmp.: 250°C, weißes Pulver.

Zur Demonstration der allmählichen Mineralisation der neuen Verbindungen und damit der Freisetzung der in ihnen enthaltenen Nährstoffe wurden die nachfolgenden Untersuchungen durchgeführt. Die gute Pflanzenverträglichkeit auch bei hohen Aufwandmengen wurden in pflanzenbaulichen Versuchen untersucht. Hierzu wurden Versuche mit Senfkeimlingen als Prüfkultur angelegt, wobei jeweils 20 Senfkörner (Sinapis alba, 'Maxi') in Plastiktöpfchen mit einem Fassungsvermögen von 660 g Limburgerhofboden (Humusboden der in Limburgerhof vorkommt) angesät wurden. Vor der Saat erfolgte eine Düngung der einzelnen Töpfchen mit steigenden Mengen der stickstoffhaltigen Prüfsubstanz. Der Boden wurde während des Versuches täglich durch Wiegen auf 60 % seiner maximalen Wasserkapazität angefeuchtet. Erfaßt wurde zu den Versuchen das Keimverhalten und während des Wachstumsverlaufes Bonituren auf Wüchsigkeit, Ausmaß von N-Mangelsymptomen ("Grünheit") und Phytotoxität vorgenommen. Ein Senf-Versuch zur Pflanzenverträglichkeit wurde je nach Wachstumsbedingungen nach 20 bis 30 Tagen beendet. Im Bedarfsfall wurde noch eine Gewichtsermittlung des Pflanzenmaterials vorgenommen, um graduelle Unterschiede zwischen den Behandlungen zu quantifizieren. Als Vergleichsbeispiele wurden analoge Versuche ohne Stickstoff, mit Harnstoff als sofort verfügbarem Stickstoff-Dünger und mit Isodur®, einem handelsüblichen Langzeitdünger, durchgeführt. Man erkennt, daß bei der höchsten Aufwandmenge von 400 mg N/Gefäß die erfindungsgemäßen Substanzen bessere Ergebnisse liefern als die Vergleichsbeispiel, insbesondere bei den Parametern Wüchsigkeit und Frischmasseertrag (FM-Ertrag).

Die Ergebnisse sind in Tabelle 3 dargestellt.

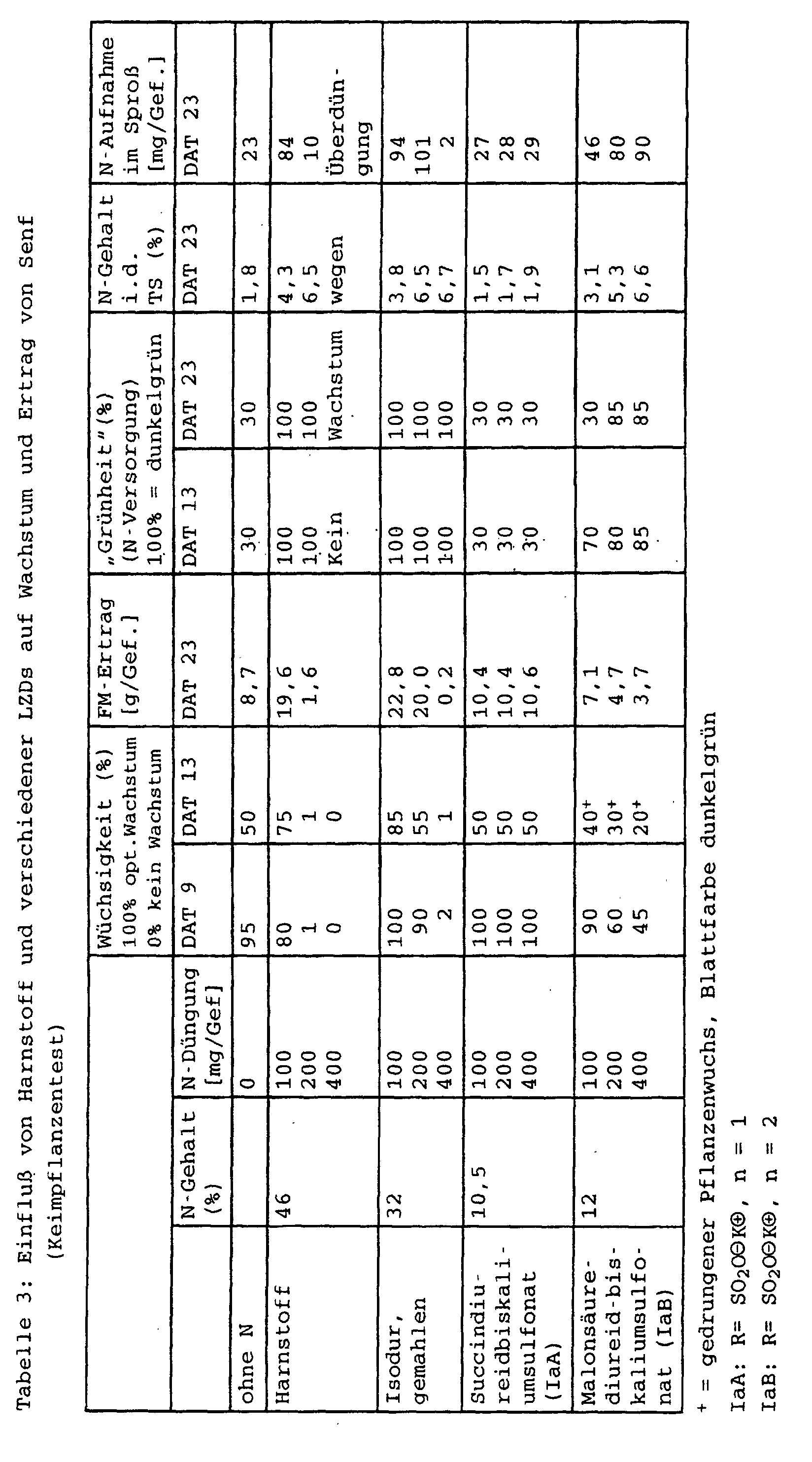

## Patentansprüche

1. Verwendung von Diureiden von Dicarbonsäuren der allgemeinen Formel I 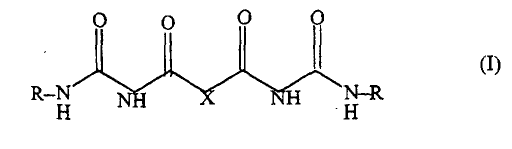 in der
R Wasserstoff oder SO₂O^{⊖}M^{⊕,}
M^{⊕} Lithium, Natrium, Kalium, Cäsium, Ammonium, Kupfer, Silber, 0,5 Eisen, 0,5 Calcium, 0,5 Magnesium, 0,5 Mangan, 0,5 Zink oder 0,5 Cobalt und
X eine gesättigte oder einfach ungesättigte, gegebenenfalls durch Sauerstoff oder NH unterbrochene geradkettige oder verzweigte, gegebenenfalls C₁- bis C₄-Alkoxy, Hydroxyl und/oder Aminogruppen tragendes C₁-bis C₈-Alkyl bedeutet,
als Langzeitdüngemittel.

2. Verwendung von Diureiden I nach Anspruch 1, **dadurch gekennzeichnet, daß** X für eine gesättigte oder einfach ungesättigte, gegebenenfalls durch Sauerstoff oder NH unterbrochene geradkettige oder verzweigte, gegebenenfalls C₁- bis C₄-Alkoxy, Hydroxyl und/oder Aminogruppen tragendes C₁- bis C₅-Alkyl steht.

3. Diureide der allgemeinen Formel Ia 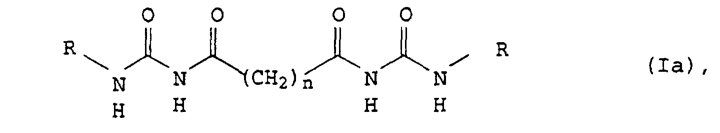 in der
R SO₂O^{⊖}M^{⊕,}
M^{⊕} Lithium, Natrium, Kalium, Cäsium, Ammonium, Kupfer, Silber, 0,5 Eisen, 0,5 Calcium, 0,5 Magnesium, 0,5 Mangan, 0,5 Zink oder 0,5 Cobalt und
n 1, 2, 3 oder 4
bedeuten.

4. Diureide Ia nach Anspruch 3, **dadurch gekennzeichnet, daß** M^{⊕} für Lithium, Natrium, Kalium, Cäsium oder Ammonium steht.

5. Diureide Ia nach einem der Ansprüche 3 oder 4, **dadurch gekennzeichnet, daß** n für 1 oder 2 steht.

6. Diureide Ia nach einem der Ansprüche 3 bis 5, **dadurch gekennzeichnet, daß** M^{⊕} für Natrium oder Kalium steht.

7. Diureide Ia nach einem der Ansprüche 3 bis 6, **dadurch gekennzeichnet, daß** M^{⊕} für Kalium steht.

8. Verfahren zur Herstellung von Diureide Ia nach einem der Ansprüche 3 bis 7, **dadurch gekennzeichnet, daß** Carbonsäurediamide der allgemeinen Formel II 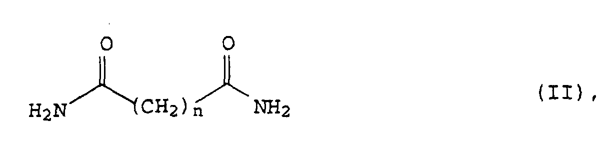 in der n für 1, 2, 3 oder 4 steht, mit Chlorsulfonylisocyanat der Formel III 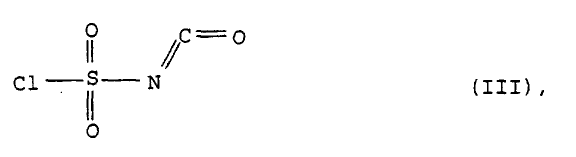 bei einer Temperatur von (-20) bis +20°C und anschließend mit wäßriger M₂CO₃-Lösung bei einer Temperatur von (-10) bis (0°C) einsetzt.

9. Verwendung der Diureide der allgemeinen Formel I bzw. Ia nach einem der Ansprüche 1 bis 8 als Dünger mit Langzeitwirkung.

10. Verwendung der Diureide der allgemeinen Formel I bzw. Ia nach einem der Ansprüche 1 bis 9 als Dünger mit Langzeitwirkung in Kombination mit anderen üblichen Düngemitteln oder Zusätzen.

11. Verwendung der Diureide der allgemeinen Formel I bzw. Ia nach Anspruch 10, **dadurch gekennzeichnet, daß** man als üblichen Düngemittel oder Zusätze Harnstoff, Ammoniumnitrat, Ammoniumsulfat, ein Ammoniumphosphat, Kaliumchlorid, Kaliumsulfat, Kaliumnitrat oder deren Gemische einsetzt.

## Claims

1. The use of diureides of dicarboxylic acids of the formula I 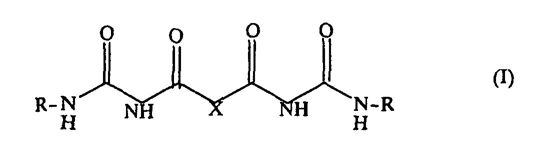 in which
R is hydrogen or SO₂O^{⊖}M^{⊕,}
M^{⊕} is lithium, sodium, potassium, cesium, ammonium, copper, silver, 0.5 iron, 0.5 calcium, 0.5 magnesium, 0.5 manganese, 0.5 zinc or 0.5 cobalt and
X is a saturated or monounsaturated, straight-chain or branched C₁- to C₈-alkyl which may be interrupted by oxygen or NH and which may have attached to it C₁- to C₄-alkoxy, hydroxyl and/or amino groups,
as slow-release fertilizers.

2. The use of diureides I as claimed in claim 1, wherein X is a saturated or monounsaturated, straight-chain or branched C₁- to C₅-alkyl which may be interrupted by oxygen or NH and which may have attached to it C₁- to C₄-alkoxy, hydroxyl and/or amino groups.

3. A diureide of the formula Ia 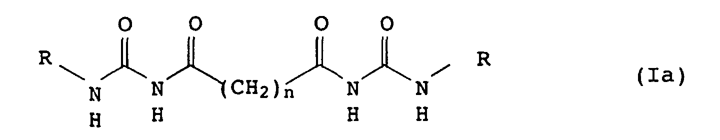 in which
R is SO₂O^{⊖}M^{⊕,}
M^{⊕} is lithium, sodium, potassium, cesium, ammonium, copper, silver, 0.5 iron, 0.5 calcium, 0.5 magnesium, 0.5 manganese, 0.5 zinc or 0.5 cobalt and
n is 1, 2, 3 or 4.

4. A diureide Ia as claimed in claim 3, wherein M^{⊕} is lithium, sodium, potassium, cesium or ammonium.

5. A diureide Ia as claimed in any of claims 3 or 4, wherein n is 1 or 2.

6. A diureide Ia as claimed in any of claims 3 to 5, wherein M^{⊕} is sodium or potassium.

7. A diureide Ia as claimed in any of claims 3 to 6, wherein M^{⊕} is potassium.

8. A process for the preparation of a diureide Ia as claimed in any of claims 3 to 7, wherein carboxylic diamides of the formula II 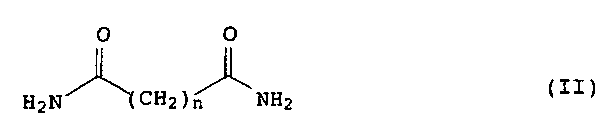 in which n is 1, 2, 3 or 4 are reacted with chlorosulfonyl isocyanate of the formula III 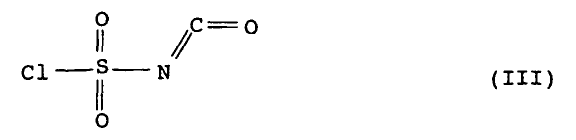 at a temperature of (-20) to +20°C and then with an aqueous M₂CO₃ solution at a temperature of (-10) to (0°C).

9. The use of a diureide of the formula I or Ia as claimed in any of claims 1 to 8 as slow-release fertilizer.

10. The use of a diureide of the formula I or Ia as claimed in any of claims 1 to 9 as slow-release fertilizer in combination with other customary fertilizers or additions.

11. The use of a diureide of the formula I or Ia as claimed in claim 10, wherein the customary fertilizers or additions employed are urea, ammonium nitrate, ammonium sulfate, an ammonium phosphate, potassium chloride, potassium sulfate, potassium nitrate or mixtures of these.

## Revendications

1. Utilisation de diuréides d'acides dicarboxyliques de formule générale I 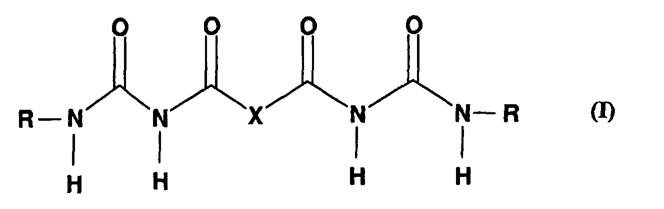 dans laquelle
R représente un atome d'hydrogène ou SO₂O^{⊖}M^{⊕}
M^{⊕} représente un ion lithium, sodium, potassium, césium, ammonium, cuivre, argent, 0,5 ion fer, 0,5 ion calcium, 0,5 ion magnésium, 0,5 ion manganèse, 0,5 ion zinc ou 0,5 ion cobalt et
X représente un groupe alkyle en C₁ à C₈ saturé ou à insaturation simple, à chaîne droite ou ramifiée, éventuellement interrompue par un atome d'oxygène ou un groupe NH, portant éventuellement des groupes alcoxy en C₁ à C₄, hydroxyle et/ou amino,
en tant qu'engrais longue durée.

2. Utilisation de diuréides I selon la revendication 1, **caractérisée en ce que** X représente un groupe alkyle en C₁ à C₅ saturé ou à insaturation simple, à chaîne droite ou ramifiée, éventuellement interrompue par un atome d'oxygène ou un groupe NH, portant éventuellement des groupes alcoxy en C₁ à C₄, hydroxyle et/ou amino.

3. Diuréides de formule générale la 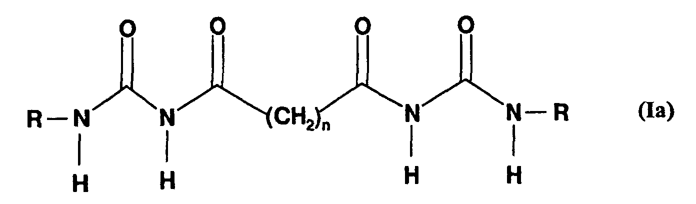 dans laquelle
R représente SO₂O^{⊖}M^{⊕},
M^{⊕} représentant un ion lithium, sodium, potassium, césium, ammonium, cuivre, argent, 0,5 ion fer, 0,5 ion calcium, 0,5 ion magnésium, 0,5 ion manganèse, 0,5 ion zinc ou 0,5 ion cobalt et
n vaut 1, 2, 3 ou 4.

4. Diuréides la selon la revendication 3, **caractérisé en ce que**, M^{⊕} représente un ion lithium, sodium, potassium, césium, ou ammonium.

5. Diuréides la selon l'une quelconque des revendications 3 ou 4, **caractérisé en ce que** n vaut 1 ou 2.

6. Diuréides la selon l'une quelconque des revendications 3 à 5, **caractérisé en ce que** M^{⊕} représente un ion sodium ou potassium.

7. Diuréides la selon l'une quelconque des revendications 3 à 6, **caractérisé en ce que** M^{⊕} représente un ion potassium.

8. Procédé de préparation de diuréides la selon l'une quelconque des revendications 3 à 7, **caractérisé en ce que** des diamides d'acide carboxylique de formule générale II 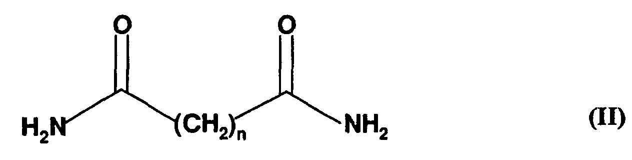 dans laquelle n vaut 1, 2, 3 ou 4, sont mis en oeuvre avec l'isocyanate de chlorosulfonyle de formule III 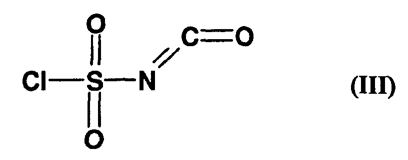 à une température de -20 à +20°C puis avec une solution aqueuse de M₂CO₃ à une température de -10 à 0°C.

9. Utilisation des diuréides de formule générale I ou la selon l'une quelconque des revendications 1-8, en tant qu'engrais ayant un effet longue durée.

10. Utilisation des diuréides de formule générale I ou la selon l'une quelconque des revendications 1-9, en tant qu'engrais ayant un effet longue durée en association avec d'autres engrais ou additifs courants.

11. Utilisation des diuréides de formule générale I ou la selon la revendication 10, **caractérisée en ce que** l'on met en oeuvre en tant qu'engrais ou additifs courants, l'urée, le nitrate d'ammonium, le sulfate d'ammonium, un phosphate d'ammonium, le chlorure de potassium, le sulfate de potassium, le nitrate de potassium ou leurs mélanges.
